# EUROPEAN PATENT APPLICATION

(11) **EP 4 782 835 A1**
(43) Date of publication of application: **29.07.2026**
(21) Application number: 24868347.6
(22) Date of filing: 20.09.2024
(51) Int. Cl.: G01N 33/543, G01N 33/02, G01N 33/12, G01N 33/569

(54) **METHOD FOR DETECTING SUBSTANCE TO BE DETECTED IN SPECIMEN, AND METHOD FOR SUPPRESSING FALSE NEGATIVES IN IMMUNOCHROMATOGRAPHIC METHOD FOR INSPECTING FOOD MATERIAL**

(30) Priority: 22.09.2023 JP 2023158396
(71) Applicant: ASAHI KASEI KABUSHIKI KAISHA, Tokyo 100-0006 (JP)
(72) Inventor: SATO, Soma, Tokyo 100-0006 (JP); FUKUSHIMA, Masakazu, Tokyo 100-0006 (JP)
(74) Representative: Forstmeyer, Dietmar
(86) International application number: PCT/JP2024/033613
(87) International publication number: WO 2025/063270

(57) **Abstract**

Provided is a method for reducing false negatives in immunochromatography. The method is a method for detecting a target analyte in a sample, the method comprising treating the sample or an extract of the sample with a compound having a functional group with an acid dissociation constant (pKa) of 3 or lower in water at 25°C, or a salt thereof; and also a method for reducing false negatives in immunochromatography or nucleic acid chromatography in which a target analyte in a sample is to be detected, the method comprising treating the sample or an extract of the sample with the compound or its salt.

## Description

### FIELD

The present invention relates to inspection of a target analyte in a food material, and particularly to a method for detecting a target analyte in a food material. The present invention also relates to a method for reducing false negatives in immunochromatography or nucleic acid chromatography. Specifically, it relates to technology for reducing false negatives in an immunochromatography method or nucleic acid chromatography method in which a target analyte in meat or seafood is to be detected.

### BACKGROUND

Immunoassay methods such as immunochromatography and latex methods are important for rapidly and reliably determining the presence of infectious disease or immune disorders, but analysis of specimens that are actually obtained from patients has been associated with the issue of false negatives, whereby a target analyte is judged to be negative despite its presence in the specimen. Since false negatives give erroneous information relating to disease, they not only delay identification of causes but can also lead to serious undesirable results, such as improper measures being taken for the disease, or increasing the gravity of the disease. Reduction of false negatives is therefore a very important goal to achieve in consideration of the main purpose of employing simple inspection methods.

PTL 1, for example, reports a method of treating a biological mucosal sample from the nasopharyngeal mucosa or saliva with piperazine-1,4-bis(2-ethanesulfonic acid (PIPES) or sodium dextran sulfate, so as to reduce false negatives which result from the presence of biological protective components in the biological mucosal sample, when detecting influenza virus from the sample, such as nasopharyngeal mucosa, saliva.

However, the main feature of the method of PTL 1 is blocking of biological protective components such as IgA using PIPES, etc., while it also fails to mention potential for application of such a method to samples other than biological mucosal samples.

PTL 2 reports on a method for reducing sedimentation of gold colloid particles to which antibodies or antigens are bound by dextran sulfate. Since gold colloid particles tend to settle, this method requires the gold colloid particle concentration to be uniform during immunoassay, and this provides a solution for the typical problem of measurement error.

However, PTL 2 also mentions nothing regarding the effect of reducing false negatives by dextran sulfate. The target of this publication is a biological sample such as blood, urine, feces or spinal fluid.

PTL 3 relates to a measuring method using immunoaggregation as the principle of measurement, wherein an aggregation promoter such as dextran sulfate or chondroitin sulfate is added. PTL 3 is characterized by using background correction, in which onlyinterference substances in a stool sample are subjected to agglutination reaction with microparticles, and the resulting background value is subtracted.

However, PTL 3 likewise mentions nothing regarding the effect of reducing false negatives by molecules such as dextran sulfate.

Incidentally, a "polyanion" is a molecule having two or more anionic groups, the molecule as a whole exhibiting a negative charge. Polyanions are widely used in immunoassays for the purpose of preventing non-specific adsorption and reducing settling of gold colloid particles, as mentioned above, but they have not been applied for target analyte analysis of food materials. Furthermore, although food materials contain a large variety of proteins, including proteins that can adversely affect antigen-antibody reaction, no reports exist regarding the relationship between polyanions and techniques for reducing false negatives that can occur due to the proteins in the food materials.

### [CITATION LIST]

### [PATENT LITERATURE]

[PTL 1] Japanese Patent No. 6116268
[PTL 2] Japanese Patent No. 5442179
[PTL 3] Japanese Patent No. 5323328

### SUMMARY

### [TECHNICAL PROBLEM]

Specific microorganisms in food materials, and especially meat and seafood, are a cause of food poisoning. Despite the importance of accurately and rapidly detecting the presence or absence of microorganisms or ensuring a hygienic state, false negatives have remained a problem due to proteins present in food materials. In particular, during inspection of a target analyte in meat or seafood using immunochromatography, etc,, the sample pad can become clogged by proteins in the meat or seafood, resulting in a false negative.

It is therefore an object of the present invention to develop a method for accurately and rapidly detecting a target analyte in a food material while reducing false negatives, and particularly reducing false negatives during inspection of a target analyte in meat or seafood using a method such as immunochromatography.

### [SOLUTION TO PROBLEM]

As a result of much diligent research directed toward solving the problems described above, the present inventors have found that by pretreating a sample with a polyanion during inspection of a target analyte in a food material by immunochromatography, etc., it is possible to reduce false negatives caused by cationic proteins or their aggregates that are characteristically present in the food material, so as to rapidly and accurately detect the target analyte in the sample.

In particular, the present inventors have found that by pretreating a sample with a polyanion during inspection of a target analyte in a food material by immunochromatography, etc., it is possible to eliminate clogging of the sample pad and thus reduce false negatives. Such clogging of the sample pad is thought to occur because cationic proteins or their aggregates, which are characteristically present in samples such as meat and seafood, bind with labeled antibodies such as negatively charged gold colloid particles, and such clogging was conjectured to be a major cause of false negatives. It was also conjectured that if the positive electrical charge of such cationic protein aggregates can be pre-neutralized, then it might be possible to inhibit binding between the cationic protein aggregates and gold colloid particles. As a result of pre-treating a sample with a polyanion capable of neutralizing positive charge, based on the aforementioned conjecture, it was possible to successfully reduce false negatives, and the present invention was thereupon completed.

Specifically, the invention provides the following.
[1] A method for detecting a target analyte in a sample, wherein the method comprises treating the sample or an extract of the sample with a compound having a functional group with an acid dissociation constant (pKa) of 3 or lower in water at 25°C, or its salt.
[2] A method for reducing false negatives in immunochromatography or nucleic acid chromatography in which a target analyte in a sample is detected, wherein the method comprises treating the sample or an extract of the sample with a compound having a functional group with an acid dissociation constant (pKa) of 3 or lower in water at 25°C, or its salt.
[3] The method according to [2], wherein the false negatives are caused by aggregates of proteins derived from the sample.
[4] The method according to [2] above, wherein the false negatives are caused when the sample is added to an immunochromatography test strip (10) which comprises a membrane provided with at least a sample pad (2), a developing unit (3) and a detector (4), in that order from the upstream end to the downstream end, due to clogging of aggregates of proteins derived from the sample that form at locations upstream from the detector.
[5] The method according to any one of [1] to [4], wherein the sample is a food material sample containing protein, or an environmental sample which comprises a food material residue containing protein.
[6] The method according to any one of [1] to [5], wherein the sample is meat or seafood.
[7] The method according to [6], wherein the meat is selected from the group consisting of chicken, pork, beef, horse meat, mutton, venison and whale, and the seafood is selected from the group consisting of tuna, salmon, sea bass, shrimp, squid, octopus and shellfish.
[8] The method according to any one of [1] to [7], wherein the compound has 3 or more phosphoric acid, sulfurous acid and/or sulfuric acid groups.
[9] The method according to any one of [1] to [8], wherein the molecular weight of the compound is in the range of 5,000 to 500,000.
[10] The method according to any one of [1] to [9], wherein the compound or its salt is polyphosphoric acid, heparin or dextran sulfate, or a salt thereof.
[11] The method according to any one of [1] to [10], wherein the compound or its salt is polyphosphoric acid or a polyphosphoric acid salt (phosphoric acid polymerization degree: 60 to 100), and the concentration of the polyphosphoric acid or polyphosphoric acid salt in the sample treatment solution obtained by treatment of the sample or an extract of the sample with the compound or its salt is in the range of 0.25 to 2.5% (w/v).
[12] The method according to any one of [1] to [10], wherein the compound or its salt is heparin or a heparin salt, and the concentration of the heparin or heparin salt in the sample treatment solution obtained by treatment of the sample or an extract of the sample with the compound or its salt is in the range of 0.0005 to 0.5% (w/v).
[13] The method according to any one of [1] to [10], wherein the compound or its salt is dextran sulfate or a dextran sulfate salt, and the concentration of the dextran sulfate or dextran sulfate salt in the sample treatment solution obtained by treatment of the sample or an extract of the sample with the compound or its salt is in the range of 0.0005 to 0.5% (w/v).
[14] The method according to any one of [1] to [13], wherein the target analyte is selected from the group consisting of allergens; components of viruses, bacteria, fungi or yeast or substances secreted by them; toxins; histamines; antibiotics; residual pesticides; and parasite antigens.
[15] The method according to any one of [1] to [14], wherein the target analyte is a bacterial component or a substance secreted by a bacterium.
[16] The method according to any one of [1] to [15], wherein the target analyte is an intracellular antigen of a bacterium.
[17] The method according to any one of [1] to [16], wherein the target analyte is a bacterial ribosomal protein.
[18] The method according to any one of [1] to [17], wherein the target analyte is Ribosomal Protein L7/L12.

### [ADVANTAGEOUS EFFECTS OF INVENTION]

According to the invention it is possible to eliminate clogging at an upstream end location from a detector such as a sample pad during inspection of a target analyte such as meat or seafood by immunochromatography, etc., and to thus reduce false negatives. Reducing false negatives allows target analytes in samples to be rapidly and accurately detected.

### BRIEF DESCRIPTION OF DRAWINGS

Fig. 1 is a schematic view of a test strip in an immunochromatography apparatus used in the method of the invention.
Fig. 2 is a photograph showing a surface image (300x) of a sample pad of a test strip before addition of a sample.
Fig. 3 is a photograph showing a surface image (300x) of a sample pad after treatment of a bacterial solution for evaluation with reaction solution B prepared in Example 5.
Fig. 4 is a photograph showing a surface image (5000x) of a sample pad after treatment of a bacterial solution for evaluation with reaction solution B prepared in Example 5.
Fig. 5 is a photograph showing a surface image (300x) of a sample pad after treatment of a bacterial solution for evaluation with reaction solution A prepared in Example 5.
Fig. 6 is a photograph showing a surface image (5000x) of a sample pad after treatment of a bacterial solution for evaluation with reaction solution A prepared in Example 5.

### DESCRIPTION OF EMBODIMENTS

The invention will now be described in greater detail by concrete embodiments thereof. However, it is to be understood that the invention is not restricted in any way to the following embodiments, and various modifications thereof may be implemented.

The first embodiment of the invention relates to a method for detecting a target analyte in a sample, the method comprising a step of treating a sample or an extract of the sample with a compound or its salt having a functional group with an acid dissociation constant (pKa) of 3 or lower in water at 25°C. The method of detection for the first embodiment is not particularly restricted, examples including immunochemical methods and spectroscopic methods, and the apparatus used for the detection method is also not particularly restricted. The method of detection for the first embodiment is particularly useful to reduce false negatives in immunochromatography or nucleic acid chromatography in which a target analyte in a sample is detected.

The second embodiment of the invention relates to a method for reducing false negatives in immunochromatography or nucleic acid chromatography in which a target analyte in a sample is detected, the method comprising a step of treating the sample or an extract of the sample with a compound or its salt having a functional group with an acid dissociation constant (pKa) of 3 or lower in water at 25°C.

The method of the first embodiment and the method of the second embodiment both have the effect of reducing false negatives caused by cationic proteins or their aggregates which are characteristically present in a sample, etc., by treating the sample with a "compound having a functional group with an acid dissociation constant (pKa) of 3 or lower in water at 25°C", or its salt.

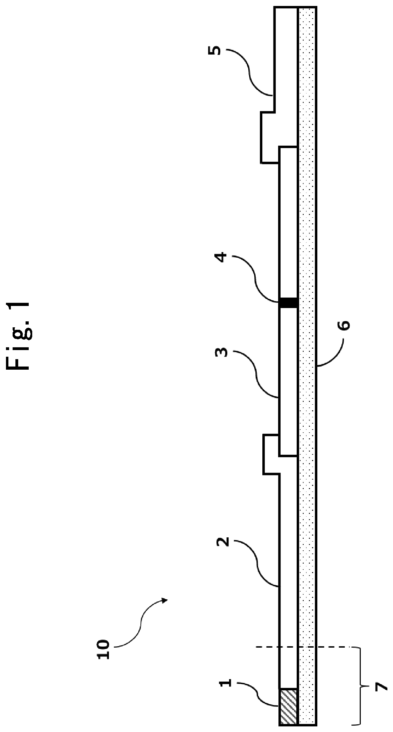

The term "false negative" means that no positive signal is generated in the assay even though the target analyte is present in the sample. In an immunochromatographic method, for example, a complex of immobilized substance-target analyte-labeling reagent is formed on a solid support when the target analyte is present in a sample, but in the case of a false negative, generation of the signal from the labeling reagent may be inhibited, either because no complexes are formed or because formation of complexes is inhibited. According to the invention, false negatives may be produced due to aggregates of sample-derived proteins, and especially aggregates of proteins believed to be cationic proteins. In particular, such false negatives can be produced when the sample is added to an immunochromatography test strip (10) (Fig. 1) which comprises a membrane provided with at least a sample pad (2), a developing unit (3) and a detector (4) in that order from the upstream direction to the downstream direction, due to clogging of aggregates of proteins derived from the sample that form at locations upstream from the detector, such as at the sample pad and/or the developing unit.

In the methods of the first embodiment and second embodiment, the "sample" is not particularly restricted, and for example, it may be a protein-containing food material sample, and preferably meat or seafood. Meat and seafood contain myofibrillar proteins, the myofibrillar proteins being composed mainly of the salt-soluble proteins myosin and actomyosin. These proteins elute by addition of salts, and initiate agglutination reaction.

Environmental samples that may comprise protein-containing food material residue are also a target of the invention. The proteins in the food material residues are preferably derived from meat or seafood.

In the method of the invention, "cationic protein" refers to a protein in which at least a portion is positively charged. The term "cationic protein" also encompasses aggregates formed by changes in its tertiary structure, etc. In practice, therefore, the "cationic protein" of the invention is expected to be primarily myosin or actomyosin.

Examples of meats include chicken, pork, beef, horse meat, mutton, goat meat, venison, whale meat, rabbit meat, boar meat, duck meat, quail meat, domesticated duck meat, pheasant meat, turkey meat, sparrow meat, ostrich meat, frog meat, terrapin meat, locust and bee meat.

Seafood is not particularly restricted and may be any aquatic animal. Examples include tuna, salmon, sea bass, salmon, trout, horse mackerel, eel, conger eel, sweetfish, monkfish, grunt, sardine, bonito, flounder, threadsail filefish, smelt-whiting, crucian carp, mackerel, shark, Japanese Spanish mackerel, pike, shishamo, sea bream, cod, loach, herring, daggertooth pike conger, puffer fish, crucian carp, yellowtail, Japanese smelt, jellyfish, mantis shrimp, sea cucumber, sea squirt and sea urchin. Seafood also includes fish eggs such as salmon roe, herring eggs and caviar.

Environmental samples that may contain food material residues are any samples obtained from an environment in contact with a food material. There are no particular restrictions on the type of environmental sample, and examples include samples obtained by wiping the surfaces of fingers, work clothes, work shoes, nail brushes, cutting boards, kitchen knives, handles, belt conveyors, packaging materials, work desks and faucets with a sampling tool (swab) such as clean cotton impregnated with liquid medium (such as water, physiological isotonic solution or ethanol), or a clean cloth, at food or beverage production facilities or food and beverage service sites, etc., or liquid samples such as tap water or well water.

Extraction of the sample can be accomplished by pulverizing and homogenizing the sample, adding an aqueous solution containing a salt such as physiological saline, and rubbing or shaking. The food material or environment surface that has been wiped with a swab, or the material wiped with the swab, may be eluted into the buffering solution to prepare a sample.

A solution (referred to as "reaction solution" in Example 2 below) of the obtained sample extract with a "compound having a functional group with an acid dissociation constant (pKa) of 3 or lower in water at 25°C" (hereinafter referred to as "compound of the invention") may be mixed in an appropriate proportion to obtain a "sample treatment solution." In some cases, the reaction solution may also contain an added compound with bacteriolytic action, or a surfactant (such as Tween^{R} 20, Tween^{R} 80, Triton^{R} X-100 or Nonidet P-40).

The "functional group having an acid dissociation constant (pKa) of 3 or lower in water at 25°C" may be a phosphoric acid group (first acid dissociation constant: 2.15), a sulfurous acid group (first acid dissociation constant: 1.86) or a sulfuric acid group (1.99) (Rika Nenpyo 2013 (desktop version), National Institutes of Natural Sciences, National Astronomical Observatory of Japan, Maruzen Publishing), p.509, 2012). The compound of the invention is not particularly restricted so long as it is a compound having a functional group selected from among phosphoric acid, sulfurous acid and sulfuric acid groups. The number of functional groups is preferably 3 or more, more preferably 4 or more, more preferably 5 or more, more preferably 6 or more, more preferably 7 or more, more preferably 8 or more, more preferably 9 or more, more preferably 10 or more, more preferably 20 or more, more preferably 30 or more, more preferably 40 or more, more preferably 50 or more and even more preferably 100 or more. Salts of a compound of the invention include sodium salts and potassium salts. The term "compound of the invention" also includes such salts unless otherwise specified.

The compound of the invention may be monomeric or polymeric so long as it is a compound having 3 or more of the aforementioned functional groups. Examples of monomeric compounds include polyhydric alcohols with 3 or more hydroxyl groups (such as glycerin), and monosaccharides.

A polymeric compound is a polymer comprising a repeating unit which includes a functional group as described above (hereunder also referred to as "polyanion" or "additive"). Examples of such polyanions include polyphosphoric acid (average phosphoric acid polymerization degree: about 60), heparin and dextran sulfate, as well as their salts. Examples of polyanion salts include sodium polyphosphate, heparin sodium and sodium dextran sulfate.

These polyanions have different molecular weights depending on the polymerization degree, but for most purposes they preferably have molecular weights of 5,000 to 500,000.

Specifically, the sodium polyphosphate may have a phosphoric acid polymerization degree of 3 or greater (a phosphoric acid number of 3 or more), but preferably it has a phosphoric acid polymerization degree of 60 to 100 and a molecular weight of 20,000 to 300,000.

Since both sodium dextran sulfate and heparin sodium are essentially sulfidized to 100%, they have 3 or more sulfuric acid groups, regardless of the polymerization degree. Examples of sodium dextran sulfates that are commercially available include those with an average molecular weight of approximately 5,000 (molecular weight of 1,000 to 9,000; number of sulfuric acid groups: about 30), of 6,500 to 10,000 (number of sulfuric acid groups: about 55), of 9,000 to 20,000 (number of sulfuric acid groups: about 94) and of approximately 500,000 (number of sulfuric acid groups: about 3,253), all of which are preferred for use. The heparin sodium used may be low molecular heparin (molecular weight: 4,000 to 6,000), but it preferably has an average molecular weight of 10,000 to 15,000.

A higher molecular weight of the polyanion can reduce false negatives even at low concentrations, and therefore a higher molecular weight is preferred for use among the commercially available products.

PIPES which has two sulfuric acid groups (piperazine-1,4-bis(2-ethanesulfonic acid)) or HEPES which has one sulfuric acid group (2-[4-(2-hydroxymethyl)piperazin-1-yl]ethanesulfonic acid) are not preferred in the method of the invention from the viewpoint of degree of sulfation.

The concentration of the compound of the invention in the sample treatment solution will differ depending on the type, but it is preferably in the following range.

When the compound of the invention is a polyphosphoric acid or polyphosphate (preferably sodium polyphosphate), the concentration of the polyphosphoric acid or polyphosphate in the sample treatment solution is preferably in the range of 0.25 to 2.5% (w/v), more preferably in the range of 0.25 to 2.4% (w/v) and even more preferably in the range of 0.25 to 1.5% (w/v).

When the compound of the invention is heparin or a heparin salt (preferably heparin sodium), the concentration of the heparin or heparin salt in the sample treatment solution is preferably in the range of 0.0005 to 0.5% (w/v), more preferably in the range of 0.0005 to 0.4% (w/v) and even more preferably in the range of 0.0005 to 0.05% (w/v).

When the compound of the invention is dextran sulfate or a dextran sulfate salt (preferably sodium dextran sulfate), the concentration of the dextran sulfate or dextran sulfate salt in the sample treatment solution is preferably in the range of 0.0005 to 0.5% (w/v), more preferably in the range of 0.0005 to 0.4% (w/v) and even more preferably in the range of 0.0005 to 0.05% (w/v).

The sample treatment solution adjusted to this concentration is then added to the sample pad in the test strip of the immunochromatography apparatus shown in Fig. 1.

The pH of the sample treatment solution is preferably 6.5 to 8.5, more preferably in the range of 6.5 to 8.0, even more preferably in the range of 7.0 to 7.5 and yet more preferably in the range of 7.4 to 7.5.

Examples of target analytes include allergens; microorganisms (such as viruses, bacteria, fungi or yeast components) or their secreted substances; toxins; histamines; antibiotics; residual pesticides; and parasite antigens.

The term "allergen" according to the invention refers primarily to a causative substance (antigen) that causes primarily dietary allergy. Examples of causative food materials include chicken, beef, pork, salmon, mackerel, shrimp, crab, abalone, squid and salmon roe (according to food labeling standards based on the Food Labeling Law Section 4, Paragraph 1).

Examples of viruses include noroviruses, hepatitis E viruses and hepatitis A viruses.

Examples of bacteria include *Salmonella* bacteria, *Campylobacter* bacteria, *Staphylococcus aureus* bacteria, enterohemorrhagic *E. coli* bacteria, pathogenic *E. coli* bacteria other than enterohemorrhagic *E. coli, Clostridium perfringens* bacteria, *Vibrio parahaemolyticus* bacteria, Clostridium botulinum bacteria, *Bacillus cereus* bacteria, *Listeria* bacteria, *Shigella* bacteria, *Yersinia enterocolitica, Vibrio vulnificus* and *Cronobacter sakazakii.*

Examples of fungi include *Mucor plumbeus, Aspergillus flavus, Aspergillus niger, Paecilomyces variotii, Fusarium oxysporum, Rhizopus stolonifer and Penicillium roqueforti.*

Examples of yeasts include baker's yeast, brewer's yeast, sake yeast, and spoilage yeast such as *Saccharomyces, Debaryomyces, Wickerhamomyces* and *Zygosaccharomyces* yeast.

Toxins include enterotoxins, endotoxins, botulinus toxin A and tetrodotoxin.

Antibiotics include penicillin, cephalosporin, tetracycline, sulfonamide, amino glycoside, aminocyclitols, macrolides, quinolones, ionophores, carbadox and nitrofurans.

According to the invention, "residual pesticide" refers to an agricultural chemical that remains in feed and becomes concentrated in the bodies of animals or fish that eat it. Examples include dicarboxyimide-based agricultural chemicals such as iprodione; dithiolane-based agricultural chemicals such as isoprothiolane; acid amide-based agricultural chemicals such as flutolanil; triazole-based agricultural chemicals such as myclobutanil and bitertanol; imidazolebased agricultural chemicals such as imazalil and triflumizole; organic halogen-based agricultural chemicals such as chlorfenapyr and chlorotalonil; carbamate-based agricultural chemicals such as carbaryl; macrolide-based agricultural chemicals such as emamectin; neonicotinoid-based agricultural chemicals such as imidacloprid and acetamiprid; and organic phosphorus-based agricultural chemicals such as malathion and isoxathione.

Parasites include *Anisakis, Nybelinia, Tentacularia, Philometroides seriolae, Pennella,* Didymozoidae, *Toxoplasma gondii, Sarcocystis hominis, S. suihominis, S. fayeri* and *Trichinella* spp.

Among these target analytes, the method of the invention is targeted at bacterial components or substances secreted by bacteria, and preferably for bacterial intracellular antigens, more preferably bacterial ribosomal proteins and even more preferably Ribosomal Protein L7/L12. Ribosomal Protein L7/L12 has a high number of copies in cells and thus allows high detection sensitivity. Moreover, as described in International Patent Publication No. WO2000/06603, it is possible to actually obtain antibodies that can distinguish specific bacteria that can cause food poisoning from other bacteria, based on genus or species.

The method of the invention may be carried out using, for example, a test strip of an immunochromatography apparatus as shown in Fig. 1 (hereunder referred to simply as "immunochromatography test strip").

The immunochromatography test strip (10) generally comprises at least a membrane made of a porous body provided with a sample pad (2), a developing unit (3) and a detector (4). A labeled antibody-attachment unit (1) with a labeled antibody that forms a complex with the target analyte is provided upstream from the sample pad. The labeled antibody is held in an elutable manner at the development start location of the developing unit. The detector is disposed at part of the downstream end of the developing unit, and it immobilizes the capturing antibody. The sample pad, the developing unit and the detector are then anchored in a line on a solid support (6), such as polyethylene. A labeled control antibody may also be added to the labeled antibody-attachment unit in addition to the labeled antibody, and a primary antibody for the control antibody that binds with the labeled control antibody may be further added to the detector. The labeled antibody-attachment unit may be disposed between the sample pad and the developing unit, or it may be disposed in the container independently from the test strip for addition of the sample treatment solution. When the labeled antibody-attachment unit is disposed between the sample pad and the developing unit, clogging by the sample (false negatives) may occur further upstream than the detector.

When the sample treatment solution is dropped onto the sample pad and the target analyte is present in the sample, the target analyte specifically binds with the labeled antibody at the developing unit, forming complexes. The complexes bind to the capture antibody at the detector while the developing unit develops in the downstream direction. At the detector, therefore, sandwich-like complexes are formed by the labeled antibody, the target analyte and the capturing antibody, and detection of the complexes allows qualitative or quantitative analysis of the target analyte.

One example of a labeling substance on a labeled antibody is gold colloid particles, which allow qualitative detection by color reaction with the gold colloid particles. It is also possible to quantitatively detect a target analyte in a sample based on the degree of color produced.

The details regarding immunochromatography test strips may be found in Japanese Patent No. 5693938, for example.

The immunochromatography apparatus can also be provided as a kit comprising a container (such as a microtube) that houses the immunochromatography test strip, and a polyanion, optionally in combination with a bacteriolytic compound.

Immunochromatography methods are largely classified into lateral flow types wherein an immunochromatography test strip is set horizontally and migration of a liquid by capillary movement (capillary force) is utilized, and flow-through types which are oriented vertically, with a liquid being passed from top to bottom by gravity and capillary force. While either a lateral flow or flow-through type may be used for the invention, a lateral flow type is preferred.

Nucleic acid chromatography is a method in which target nucleic acid is extracted from a sample, the target nucleic acid is used as template for PCR amplification reaction with a tagged special primer, the amplified target nucleic acid being developed into a dedicated strip (nucleic acid chromatographic chip), and the presence or absence of target DNA being converted into bands and visually assessed (for example, Japanese Unexamined Patent Publication No. 2014-82977).

The present invention will now be described in greater detail by examples, with the understanding that the invention is not limited to these examples.

### EXAMPLES

### Example 1 Materials and preparation method

### 1. Construction of labeling antibody and capturing antibody

*Staphylococcus aureus* was used as the immunogen bacteria. Antibodies for *Staphylococcus aureus* ribosomal protein L7/L12 were prepared with reference to the method described in International Patent Publication No. WO2000/06603.

Specifically, *E. coli* transformed with an expression vector in which DNA coding for the full length of the amino acid sequence of *Staphylococcus aureus* ribosomal protein L7/L12 was incorporated, was cultured using LB medium, and the tag sequence from the expression vector was purified as a fusion protein using an affinity column. The L7/L12 full-length protein was used as immunogen and prepared to an immunogen concentration of 0.4 mg/mL with PBS according to an established method for obtaining hybridomas, and then Freund's adjuvant was added in the same amount and mice were immunized 4 times with an immunogen content of 50 µg/immunization. After confirming serum antibody titer increase by test blood collection, the mouse spleen cells were extracted. The extracted mouse spleen cells were fused with myeloma cells to obtain various hybridomas.

The obtained hybridomas were cultured in HAT medium and screened based on the antibodies in the culture supernatants. The screening was carried out by ELISA, and two different hybridomas were selected to produce antibodies that exhibit specific reactivity for *Staphylococcus aureus.*

Each selected hybridoma was cultured in TIL MediaI medium containing 10% added fetal bovine serum (FBS), following an established method for monoclonal antibody production, and intraperitoneally administered to mice, and the ascites fluid was collected. The collected ascites fluid was centrifuged to separate the suspended matter and erythrocytes, and then filtered with a filter having a mesh opening of 0.45 µm. The obtained filtrate was passed through a Protein G column to adsorb the antibody, thus purifying the obtained antibody from the mouse ascites fluid.

### 2. Fabrication of immunochromatography test strip

The obtained antibodies were used as capturing antibody and labeling antibody to fabricate an immunochromatography test strip.

### (1) Immunochromatography development membrane support

The capturing antibody was mixed with 100 mM sodium phosphate buffer to 1.5 mg/mL each, and trehalose was added at 3% (v/v) to prepare a solution. The obtained solution was coated onto a commercially available nitrocellulose membrane cut to a width of 2.5 cm and a length of 15 cm, as a single line with a liquid volume of 1 µL per 1 cm², and dried to obtain an immunochromatography development membrane support.

### (2) Preparation of gold colloid-labeling antibody and gold colloid-labeling antibody-attached member

A 1/10 amount of labeling antibody was added to and mixed with a commercially available gold colloid solution (particle size: 60 nm), to prepare a solution with an antibody concentration of 0.1 mg/mL. The solution was allowed to stand at room temperature for 30 minutes for bonding of the antibody to the gold colloid particle surfaces. Then, BSA solution was added to the gold colloid solution to a final concentration of 0.1% for blocking, to prepare a gold colloid-labeling antibody solution. The antibody solution was used to soak a commercially available glass fiber sheet and then dried to prepare a gold colloid-labeling antibody-attached member.

### (3) Fabrication of immunochromatography test strip

In addition to the immunochromatography development membrane support fabricated in (1) and the gold colloid-labeling antibody-attached member fabricated in (2), there were also prepared a polyester fiber nonwoven fabric as a member for addition of the sample, and a filter paper as an absorption material. The members were attached to a commercially available polyethylene substrate and then cut to a width of 5 mm to fabricate an immunochromatography test strip having the same construction as shown in Fig. 1.

### Example 2 Inhibiting clogging of gold colloid with different reaction solution compositions

### (1) Preparation of bacterial solutions for evaluation

With beef (minced), pork (minced), chicken (minced), tuna meat (sashimi) and salmon meat (sashimi) selected as food materials, 5 g of each food material was weighed out and placed in a Stomacher^{R} bag containing 45 ml physiological saline, and the mixture was rubbed for 60 seconds to obtain an extract of each food material. The selected bacteria to be detected were *Staphylococcus aureus,* which were prepared to 1 × 10⁵ cfu/ml using different food material extracts or physiological saline.

### (2) Preparation of reaction mixtures

To 0.1 M Tris-HCl buffer (pH 7.4) there was added 5 µg/ml lysostaphin for bacteriolysis of *Staphylococcus aureus,* and a mother liquor was prepared by adding 0.2% Tween^{R} 20 for immunochromatography development, after which each of the additives listed in Table 1 were added to prepare reaction mixtures under the conditions shown in Table 1.

Table 1 also shows examples of using sodium citrate under condition (1-6), PIPES under condition (1-7) and HEPES under condition (1-8) for comparison. The concentration of each additive was 0.5% (w/v).

**[Table 1]**

| Table 1 Reaction mixture composition conditions | |
|---|---|
| Condition | Additive |
| 1-1 | - |
| 1-2 | Sodium dextran sulfate |
| 1-3 | Sodium polyphosphate |
| 1-4 | Heparin sodium |
| 1-5 | Carboxymethyldextran |
| 1-6 | Sodium citrate |
| 1-7 | PIPES (1,4-piperazinediethanesulfonic acid) |
| 1-8 | HEPES (4-(2-hydroxyethyl)-1-piperazineethanesulfonic acid) |

### (3) Evaluation with immunochromatography test strip

The bacterial solutions for evaluation and each of the reaction mixtures were mixed in a proportion of 1:1, and then the immunochromatography test strip prepared in Example 1 was inserted into the mixture and the presence or absence of detection by antigen-antibody reaction was evaluated.

The evaluation was conducted by visually judging the degree of redness of the test line sections produced by gold colloid particles. A positive visual result was indicated as "+", a weak positive result was indicated as "±", and a negative visual result with gold colloid particle clogging in the sample pad was indicated as "-". The results are shown in Table 2.

**[Table 2]**

| Table 2 Judgment results under dirrerent reaction mixture composition conditions | | | | | | |
|---|---|---|---|---|---|---|
| Condition | Physiological saline | Beef | Pork | Chicken | Tuna | Salmon |
| 1-1 | + | - | - | - | - | - |
| 1-2 | + | + | + | + | + | + |
| 1-3 | + | + | + | + | + | + |
| 1-4 | + | + | + | + | + | + |
| 1-5 | + | - | - | - | - | - |
| 1-6 | + | - | - | - | - | - |
| 1-7 | + | - | - | - | - | - |
| 1-8 | + | - | - | - | - | - |

Under the reaction mixture condition (1-1) with the mother liquor alone, insertion of the immunochromatography test strip into the different food material extracts resulted in gold colloid particle clogging in the sample pad, and a negative visual result. This result is attributed to inhibited migration of the gold colloid particle solution by aggregates of proteins thought to be cationic proteins in the food material extracts. On the other hand, under conditions (1-2) to (1-4) in which different polyanions were added, no clogging of the gold colloid particles was observed in the sample pad with any of the food material extracts, and positive visual results were obtained. Also, under condition (1-5) in which a polyanion with a carboxymethyl group was added, under condition (1-6) in which citric acid was added or under conditions (1-7) and (1-8) in which a compound with 2 or fewer sulfuric acid groups was added, gold colloid particle clogging occurred in the sample pad and a negative visual result was obtained, similar to reaction mixture condition (1-1) with the mother liquor alone.

These results indicated that using a specific polyanion can reduce false negatives caused by proteins thought to be cationic proteins.

### Example 3 Examination of polyanion concentration

### (1) Preparation of bacterial solutions for evaluation

With beef (minced), pork (minced), chicken (minced), tuna meat (sashimi) and salmon meat (sashimi) selected as food materials, 5 g of each food material was weighed out and placed in a Stomacher^{R} bag containing 45 ml physiological saline, and the mixture was rubbed for 60 seconds to obtain an extract of each food material. The selected bacteria to be detected were *Staphylococcus aureus,* prepared to 1 × 10⁵ cfu/ml using different food material extracts or physiological saline.

### (2) Preparation of reaction mixtures

To 0.1 M Tris-HCl buffer (pH 7.4) there was added 5 µg/ml lysostaphin for bacteriolysis of *Staphylococcus aureus,* with further addition of 0.2% Tween^{R} 20 for immunochromatography development, for use as the mother liquor, and reaction mixtures were prepared comprising 0.0001 to 5% (w/v) of the different additives.

The sodium dextran sulfate and heparin sodium were prepared to a range of 0.0001 to 1% (w/v), and sodium polyphosphate and carboxymethyldextran were prepared to a range of 0.0001 to 5% (w/v).

### (3) Evaluation with immunochromatography test strip

The bacterial solutions for evaluation and each of the reaction mixtures were mixed in a proportion of 1:1, and then an immunochromatography test strip was inserted into the mixture and the presence or absence of detection by antigen-antibody reaction was evaluated.

The evaluation was conducted by visually judging the degree of redness of the test line sections produced by gold colloid particles. A positive visual result was indicated as "+", a weak positive visual result was indicated as "±", and a negative visual result with gold colloid particle clogging in the sample pad was indicated as "-". The results are shown in Table 3.

**[Table 3]**

| Table 3 Concentration conditions and judgment results for different sample treatment solutions | | | | | | | | |
|---|---|---|---|---|---|---|---|---|
| Condition | Sample treatment solution | | Visual judgment | | | | | |
| | Additive | Additive concentration | Physiological saline | Beef | Pork | Chicken | Tuna | Salmon |
| 2-1 | Sodium dextran sulfate | 0.00005% | + | - | - | - | - | - |
| 2-2 | | 0.0005% | + | + | + | + | + | + |
| 2-3 | | 0.005% | + | + | + | + | + | + |
| 2-4 | | 0.05% | + | + | + | + | + | + |
| 2-5 | | 0.5% | + | + | + | + | + | + |
| 2-6 | Sodium polyphosphate | 0.00005% | + | - | - | - | - | - |
| 2-7 | | 0.0005% | + | - | - | - | - | - |
| 2-8 | | 0.005% | + | - | - | - | - | - |
| 2-9 | | 0.05% | + | - | - | - | - | - |
| 2-10 | | 0.25% | + | + | + | + | + | + |
| 2-11 | | 0.5% | + | + | + | + | + | + |
| 2-12 | | 1.5% | + | + | + | + | + | + |
| 2-13 | | 2.5% | + | + | + | + | + | + |
| 2-14 | Heparin sodium | 0.00005% | + | - | - | - | - | - |
| 2-15 | | 0.0005% | + | + | + | + | + | + |
| 2-16 | | 0.005% | + | + | + | + | + | + |
| 2-17 | | 0.05% | + | + | + | + | + | + |
| 2-18 | | 0.5% | + | + | + | + | + | + |
| 2-19 | Carboxymethyldextran | 0.00005% | + | - | - | - | - | - |
| 2-20 | | 0.0005% | + | - | - | - | - | - |
| 2-21 | | 0.005% | + | - | - | - | - | - |
| 2-22 | | 0.05% | + | - | - | - | - | - |
| 2-23 | | 0.5% | + | - | - | - | - | - |
| 2-24 | | 1.5% | + | - | - | - | - | - |
| 2-25 | | 2.5% | + | - | - | - | - | - |

When sodium dextran sulfate was used as the additive, an inhibiting effect against gold colloid particle clogging was confirmed under the additive concentration in the sample treatment solution was 0.0005% (w/v) or higher, with the test line strength by antigen-antibody reaction also being sufficiently high. On the other hand, when the additive concentration in the sample treatment solution was 0.5% (w/v) or higher, the test line strength was reduced (weak positive visual result), even without clogging of the gold colloid particles. This confirmed that a sodium dextran sulfate concentration in the range of 0.0005 to 0.5% (w/v) in the sample treatment solution is suitable.

When sodium polyphosphate was used as the additive, an inhibiting effect against gold colloid particle clogging was confirmed under the additive concentration in the sample treatment solution was 0.025% (w/v) or higher, with the test line strength by antigen-antibody reaction also being sufficiently high. On the other hand, when the additive concentration in the sample treatment solution was 2.5% (w/v) or higher, the test line strength was reduced (weak positive visual result), even without clogging of the gold colloid particles. This confirmed that a sodium polyphosphate concentration in the range of 0.25 to 2.5% (w/v) in the sample treatment solution is suitable.

When heparin sodium was used as the additive, an inhibiting effect against gold colloid particle clogging was confirmed under the additive concentration in the sample treatment solution was 0.0005% (w/v) or higher, with the test line strength by antigen-antibody reaction also being sufficiently high. On the other hand, when the additive concentration in the sample treatment solution was 0.5% (w/v) or higher, the test line strength was reduced (weak positive visual result), even without clogging of the gold colloid particles. This confirmed that a heparin sodium concentration in the range of 0.0005 to 0.5% (w/v) in the sample treatment solution is suitable.

When carboxymethyldextran was used as the additive, gold colloid particle clogging occurred under all of the additive concentration conditions, with a negative visual result.

### Example 4 Examination of polyanion pH

### (1) Preparation of bacterial solutions for evaluation

With beef (minced), pork (minced), chicken (minced), tuna meat (sashimi) and salmon meat (sashimi) selected as food materials, 5 g of each food material was weighed out and placed in a Stomacher^{R} bag containing 45 ml physiological saline, and the mixture was rubbed for 60 seconds to obtain an extract of each food material. The selected bacteria to be detected were *Staphylococcus aureus,* prepared to 1 × 10⁵ cfu/ml using different food material extracts or physiological saline.

### (2) Preparation of reaction mixtures

To a 0.1 M Tris-HCl (or MES-NaOH) buffer there was added 5 µg/ml of lysostaphin for bacteriolysis of *Staphylococcus aureus,* and a mother liquor was prepared by adding 0.2% Tween^{R} 20 for immunochromatography development, adjusting each of the additives to the prescribed concentration and then adjusting the pH, to prepare reaction mixtures with different pH values (5.5 to 8.5).

Sodium dextran sulfate and heparin sodium were prepared to 0.01% (w/v), and sodium polyphosphate was prepared to 1% (w/v).

### (3) Evaluation with immunochromatography test strip

The bacterial solutions for evaluation and each of the reaction mixtures were mixed in a proportion of 1:1, and then an immunochromatography test strip was inserted into the mixture and the presence or absence of detection by antigen-antibody reaction was evaluated.

The evaluation was conducted by visually judging the degree of redness of the test line sections produced by gold colloid particles. A positive visual result was indicated as "+", a weak positive result was indicated as "±", a negative visual result with gold colloid particle clogging in the sample pad was indicated as "-", and a negative visual result without gold colloid particle clogging in the sample pad was indicated as "--". The results are shown in Table 4.

**[Table 4]**

| Table 4 pH conditions and judgment results for different sample treatment solutions | | | | | | | | |
|---|---|---|---|---|---|---|---|---|
| Condition | Sample treatment solution | | Visual judgment | | | | | |
| | Additive | pH | Physiological saline | Beef | Pork | Chicken | Tuna | Salmon |
| 3-1 | - | 8.5 | + | - | - | - | - | - |
| 3-2 | | 8.0 | + | - | - | - | - | - |
| 3-3 | | 7.5 | + | - | - | - | - | - |
| 3-4 | | 7.0 | + | - | - | - | - | - |
| 3-5 | | 6.5 | ± | - | - | - | - | - |
| 3-6 | | 6.0 | ± | - | - | - | - | - |
| 3-7 | | 5.5 | -- | - | - | - | - | - |
| 3-8 | Sodium dextran sulfate | 8.5 | + | + | + | + | + | + |
| 3-9 | | 8.0 | + | + | + | + | + | + |
| 3-10 | | 7.5 | + | + | + | + | + | + |
| 3-11 | | 7.0 | + | + | + | + | + | + |
| 3-12 | | 6.5 | + | + | + | + | + | ± |
| 3-13 | | 6.0 | ± | - | - | - | - | - |
| 3-14 | | 5.5 | -- | - | - | - | - | - |
| 3-15 | Sodium polyphosphate | 8.5 | + | + | + | + | + | + |
| 3-16 | | 8.0 | + | + | + | + | + | + |
| 3-17 | | 7.5 | + | + | + | + | + | + |
| 3-18 | | 7.0 | + | + | + | + | + | + |
| 3-19 | | 6.5 | + | + | + | + | + | ± |
| 3-20 | | 6.0 | ± | - | - | - | - | - |
| 3-21 | | 5.5 | - | - | - | - | - | - |
| 3-22 | Heparin sodium | 8.5 | + | + | + | + | + | + |
| 3-23 | | 8.0 | + | + | + | + | + | + |
| 3-24 | | 7.5 | + | + | + | + | + | + |
| 3-25 | | 7.0 | + | + | + | + | + | + |
| 3-26 | | 6.5 | + | + | + | + | + | ± |
| 3-27 | | 6.0 | ± | - | - | - | - | - |
| 3-28 | | 5.5 | -- | - | - | - | - | - |

The pH values in Table 4 are the pH values of the sample treatment solutions.

Table 4 confirms that a sufficient inhibiting effect against gold colloid particle clogging is exhibited by limiting the pH of the sample treatment solution to the range of 6.5 to 8.5, under polyanion-added conditions (3-8) to (3-28).

On the other hand, it was also confirmed that with conditions (3-1) to (3-7) in which a polyanion was not added, gold colloid particle clogging occurred in the food material extracts under all of the pH conditions.

While an inhibiting effect against gold colloid particle clogging was confirmed even without addition of a polyanion under conditions where the pH was higher than 8.5, the detection sensitivity also tended to be reduced.

### Example 5 Observation of sample pad surface with gold colloid particle clogging

### (1) Preparation of bacterial solutions for evaluation

Chicken (minced meat) was selected as the food material, and 5 g was weighed out and placed in a Stomacher^{R} bag containing 45 ml physiological saline and rubbed for 60 seconds to obtain a food material extract. The selected bacteria to be detected were *Staphylococcus aureus,* and prepared to 1 × 10⁵ cfu/ml using the food material extract.

### (2) Preparation of reaction mixtures

To 0.1 M Tris-HCl buffer (pH 7.4) there was added 5 µg/ml lysostaphin for bacteriolysis of *Staphylococcus aureus,* and a mother liquor was prepared by adding 0.2% Tween^{R} 20 for immunochromatography development, to prepare "reaction mixture A" with addition of 0.5% (w/v) sodium dextran sulfate, and "reaction mixture B" with the mother liquor alone.

### (3) Observation of sample pad surface after evaluation of immunochromatography test strip

The bacterial solutions for evaluation and each reaction solution were mixed in a proportion of 1:1, and after inserting an immunochromatography test strip into the mixture, a sample pad was cut out at a location in contact with the liquid level (indicated as 7 in Fig. 1), and the surface of the sample pad was observed with a scanning electron microscope (FlexSEM1000, Hitachi High-Technologies Corp.).

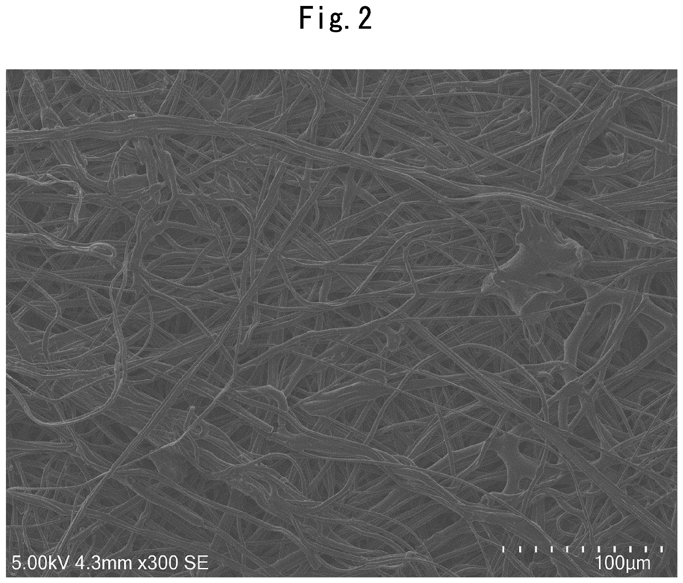

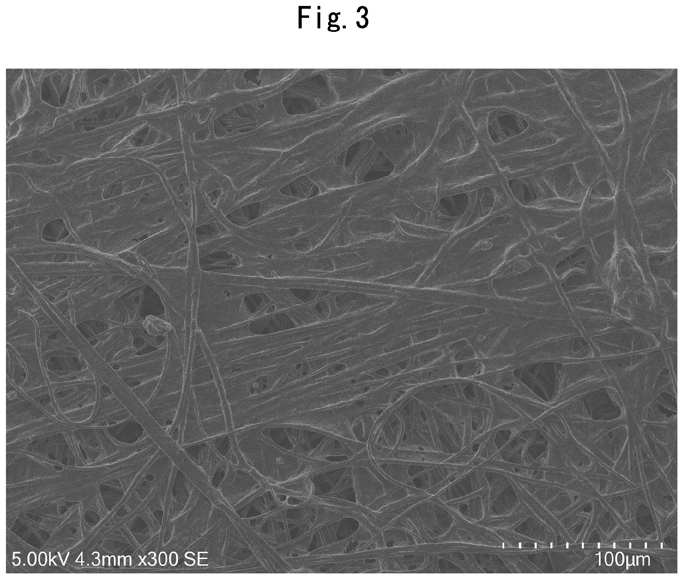

When the bacterial solution for evaluation was treated with reaction mixture A, no clogging of gold colloid particles was observed in the sample pad. On the other hand, when the bacterial solution for evaluation was treated with reaction mixture B, reddish aggregates were observed in the sample pad, confirming clogging of the gold colloid particles. Fig. 2 shows a surface image of the sample pad before addition of the sample, and Fig. 3 shows a surface image of the sample pad after treatment of the bacterial solution for evaluation with reaction mixture B (300x for each). The images confirmed that treatment of the bacterial solution for evaluation with reaction mixture B results in more clogging between the fibers of the sample pad with what appears to be aggregates, compared to before addition of the sample.

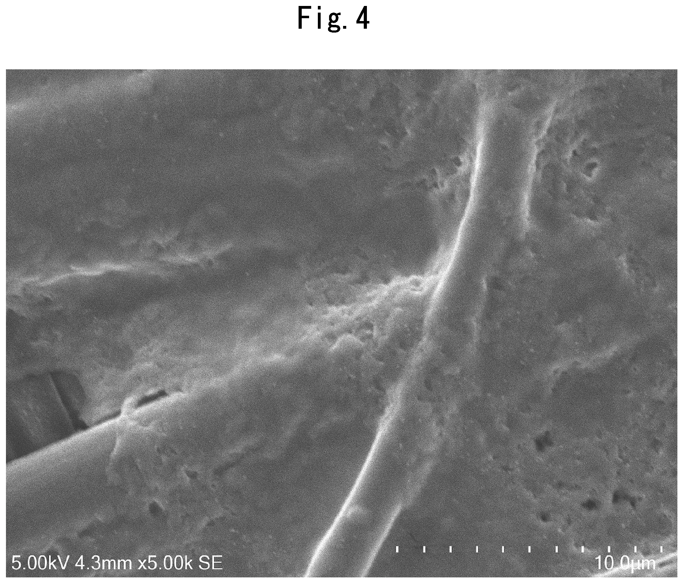

Fig. 4 shows a surface image of a sample pad after treatment of the bacterial solution for evaluation with reaction mixture B at high magnification (5000x). This image shows adhesion of particulate substances on the aggregate surfaces. The aggregates were aggregates of proteins presumed to be cationic proteins, and the particulate substances were presumed to be gold colloid particles.

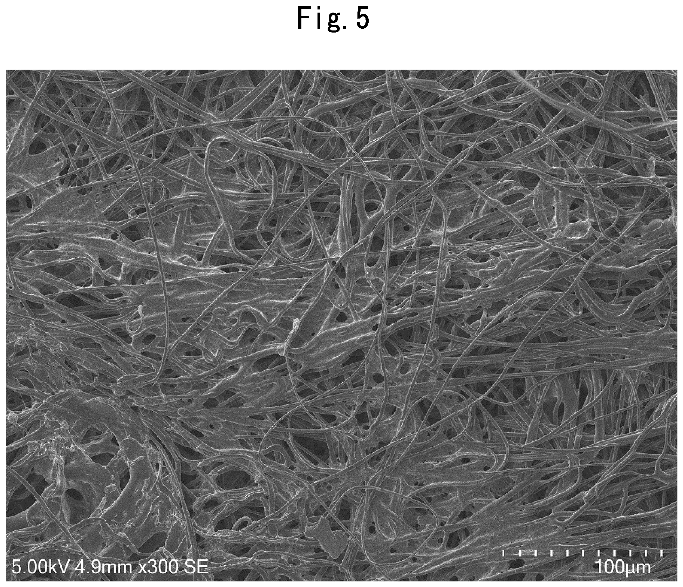

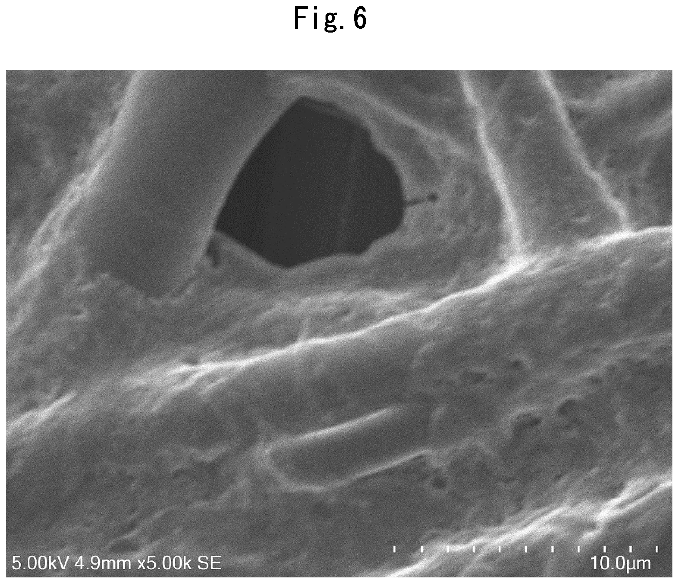

Figs. 5 and 6 show surface images of a sample pad after treatment of the bacterial solution for evaluation with reaction mixture A. This image shows clogging between the fibers of the sample pad with what appears to be aggregates (although the degree of aggregation was lower than by treatment with reaction mixture B), but it was confirmed that no particulate substances were adhering to the aggregate surfaces.

This suggests that when the bacterial solution for evaluation was treated with a polyanion such as sodium dextran sulfate, (i) the polyanion reduced secondary aggregation formation from the aggregates of proteins presumed to be cationic proteins, and (ii) the polyanion masked aggregates of proteins presumed to be cationic proteins which were already present, thereby neutralizing the positive electrical charge of the protein aggregates, and blocking binding between the protein aggregates and the negatively charged gold colloid particles, which eliminated blocking of the sample pad. Presumably, elimination of clogging resulted in formation of the expected antigen-antibody reaction complexes, thereby reducing false negatives.

### INDUSTRIAL APPLICABILITY

The method of the invention can be used for rapid and accurate examination of target analytes in meat or seafood.

### REFERENCE SIGNS LIST

10 Immunochromatography apparatus test strip
1 Labeled antibody-attachment unit
2 Sample pad
3 Developing unit
4 Detector
5 Absorbent pad
6 Solid support
7 Liquid surface height

## Claims

1. A method for detecting a target analyte in a sample, wherein the method comprises treating the sample or an extract of the sample with a compound having a functional group with an acid dissociation constant (pKa) of 3 or lower in water at 25°C, or its salt.

2. A method for reducing false negatives in immunochromatography or nucleic acid chromatography in which a target analyte in a sample is detected, wherein the method comprises treating the sample or an extract of the sample with a compound having a functional group with an acid dissociation constant (pKa) of 3 or lower in water at 25°C, or its salt.

3. The method according to claim 2, wherein the false negatives are caused by aggregates of proteins derived from the sample.

4. The method according to claim 2, wherein the false negatives are caused when the sample is added to an immunochromatography test strip (10) which comprises a membrane provided with at least a sample pad (2), a developing unit (3) and a detector (4), in that order from the upstream end to the downstream end, due to clogging of aggregates of proteins derived from the sample that form at locations upstream from the detector.

5. The method according to claim 1 or 2, wherein the sample is a food material sample containing protein, or an environmental sample which comprises a food material residue containing protein.

6. The method according to claim 1 or 2, wherein the sample is meat or seafood.

7. The method according to claim 6, wherein the meat is selected from the group consisting of chicken, pork, beef, horse meat, mutton, venison and whale, and the seafood is selected from the group consisting of tuna, salmon, sea bass, shrimp, squid, octopus and shellfish.

8. The method according to claim 1 or 2, wherein the compound has 3 or more phosphoric acid, sulfurous acid and/or sulfuric acid groups.

9. The method according to claim 1 or 2, wherein the molecular weight of the compound is in the range of 5,000 to 500,000.

10. The method according to claim 1 or 2, wherein the compound or its salt is polyphosphoric acid, heparin or dextran sulfate, or a salt thereof.

11. The method according to claim 1 or 2, wherein the compound or its salt is polyphosphoric acid or a polyphosphoric acid salt (phosphoric acid polymerization degree: 60 to 100), and the concentration of the polyphosphoric acid or polyphosphoric acid salt in the sample treatment solution obtained by treatment of the sample or an extract of the sample with the compound or its salt is in the range of 0.25 to 2.5% (w/v).

12. The method according to claim 1 or 2, wherein the compound or its salt is heparin or a heparin salt, and the concentration of the heparin or heparin salt in the sample treatment solution obtained by treatment of the sample or an extract of the sample with the compound or its salt is in the range of 0.0005 to 0.5% (w/v).

13. The method according to claim 1 or 2, wherein the compound or its salt is dextran sulfate or a dextran sulfate salt, and the concentration of the dextran sulfate or dextran sulfate salt in the sample treatment solution obtained by treatment of the sample or an extract of the sample with the compound or its salt is in the range of 0.0005 to 0.5% (w/v).

14. The method according to claim 1 or 2, wherein the target analyte is selected from the group consisting of allergens; components of viruses, bacteria, fungi or yeast or, substances secreted by them; toxins; histamines; antibiotics; residual pesticides; and parasite antigens.

15. The method according to claim 1 or 2, wherein the target analyte is a bacterial component or a substance secreted by a bacterium.

16. The method according to claim 1 or 2, wherein the target analyte is an intracellular antigen of a bacterium.

17. The method according to claim 1 or 2, wherein the target analyte is a bacterial ribosomal protein.

18. The method according to claim 1 or 2, wherein the target analyte is Ribosomal Protein L7/L12.
